**Europäisches Patentamt**

**European Patent Office**

**Office européen des brevets**

(19)

(11) Veröffentlichungsnummer : **0 171 632 B1**

# EUROPÄISCHE PATENTSCHRIFT

(12)

(45) Veröffentlichungstag der Patentschrift :
**04.03.87**

(51) Int. Cl.⁴ : **C 07 C 29/15, C 07 C 31/20**

(21) Anmeldenummer : **85108901.1**

(22) Anmeldetag : **16.07.85**

(54) Verfahren zur Herstellung von Ethylenglycol.

(30) Priorität : **24.07.84 DE 3427138**

(43) Veröffentlichungstag der Anmeldung :
**19.02.86 Patentblatt 86/08**

(45) Bekanntmachung des Hinweises auf die Patenterteilung : **04.03.87 Patentblatt 87/10**

(84) Benannte Vertragsstaaten :
**BE DE FR GB IT NL**

(56) Entgegenhaltungen :
**US-A- 3 974 259**

(73) Patentinhaber : **BASF Aktiengesellschaft**
**Carl-Bosch-Strasse 38**
**D-6700 Ludwigshafen (DE)**

(72) Erfinder : **Bertleff, Werner, Dr.**
**Neuzenlache 3**
**D-6806 Viernheim (DE)**
Erfinder : **Mueller, Franz-Josef, Dr.**
**Mueller-Thurgau-Weg 1**
**D-6706 Wachenheim (DE)**
Erfinder : **Kummer, Rudolf, Dr.**
**Kreuzstrasse 6**
**D-6710 Frankenthal (DE)**
Erfinder : **Harder, Wolfgang, Dr.**
**Bergwaldstrasse 16**
**D-6940 Weinheim (DE)**

EP 0 171 632 B1

## Beschreibung

Die vorliegende Erfindung betrifft ein verbessertes Verfahren zur Herstellung von Ethylenglycol aus Kohlenmonoxid und Wasserstoff unter erhöhtem Druck und bei erhöhter Temperatur in Gegenwart von Rhodium enthaltenden Katalysatoren.

Dieses Verfahren ist, sieht man von der erfindungsgemäßen Verbesserung ab, aus zahlreichen Veröffentlichungen allgemein bekannt. Speziell sind für diese sogenannte Direktsynthese von niederen Alkoholen aus CO und $H_2$, bei der neben dem hauptsächlich erwünschten Ethylenglycol u. a. Methanol und Ethanol gebildet werden, auch schon solche Katalysatoren empfohlen worden, die zwei oder drei verschiedene Metalle enthalten, darunter Rhodium gemeinsam mit Cobalt in den folgenden Carbonylkomplexen « Clustern »

$CoRh_{12}(CO)_{30}$
$[Co_yRh_{12-y}(CO)_{30}]_3M_2$
$y = 1-11$ ; M = dreiwertiges Metall
$[Co_yRh_{12-y}(CO)_{30}]M_2'$
$M' = $ u. a. einwertiges Rhodium

gemäß den US-Patentschriften 3 878 290, 3 929 969 bzw. 3 974 259.

Wie aus diesen Formeln hervorgeht, beträgt hierbei das Molverhältnis von Rhodium zu Cobalt höchstens 13 : 1.

Die mit diesen Clustern unter verschiedenen Bedingungen erzielbaren Ausbeuten an Ethylenglycol vermögen jedoch nicht zu befriedigen, weshalb der Erfindung die Aufgabe zugrunde lag, diese Ausbeuten zu erhöhen.

Demgemäß wurde ein verbessertes Verfahren zur Herstellung von Ethylenglycol aus Kohlenmonoxid und Wasserstoff unter erhöhtem Druck und bei erhöhter Temperatur in Gegenwart von Rhodium enthaltenden Katalysatoren gefunden, welches dadurch gekennzeichnet ist, daß man zusätzlich zu den Rhodiumkatalysatoren Cobaltkatalysatoren verwendet, wobei das Molverhältnis von Rhodium zu Cobalt 20 : 1 bis 60 : 1 beträgt.

Da es nicht erforderlich ist, daß Rhodium und Cobalt einen definierten Carbonylkomplex bilden, können beide Metalle getrennt eingesetzt werden, und zwar entweder in Form ihrer reinen Carbonylkomplexe wie $Co_2(CO)_8$, $Co_4(CO)_{12}$, $Rh_4(CO)_{12}$ und $Rh_6(CO)_{16}$, in Form sonstiger Komplexe, z. B. mit Acetylaceton oder in Form von Salzen oder Oxiden wie Cobaltacetat, Cobaltcarbonat, Cobaltnitrat, Rhodiumchlorid, Rhodiumacetat und Rhodiumoxid, da sich die aktiven Komplexe jeweils *in situ* bilden.

Auf welche Weise sich das Ethylenglycol aus den Bausteinen CO und $H_2$ im einzelnen bildet, ist nicht genau bekannt, jedoch kann es als gesichert gelten, daß hierbei mehrere Reaktionen beteiligt sind, wobei eine modellhafte Reaktionsfolge in der Bildung von Formaldehyd oder eines Metallformylkomplexes, in der Oxierung zum Glycolaldehyd und in der Hydrierung dieses Aldehyds bestehen könnte. Von jeder dieser Stufen aus sind unerwünschte Nebenreaktionen möglich, z. B. die Hydrierung des Formaldehyds zum Methanol sowie andere Reaktionen (z. B. Homologisierung), die zum Ethanol führen, wobei ein einziger Katalysator vermutlich nicht in der Lage ist, die gesamte Reaktionsfolge im gewünschten Sinne zu begünstigen. Dies jedoch wird durch die erfindungsgemäße Verwendung zweier verschiedener Katalysatoren, nämlich solchen auf Basis von Rhodium und solchen auf Basis von Cobalt, zufriedenstellend bewirkt, und zwar im Bereich der angegebenen Molverhältnisse, wobei sich ein Rh/Co-Verhältnis von 25 : 1 bis 30 : 1 meistens besonders empfiehlt.

Daß die Ethylenglycol-Ausbeute auch noch von anderen Reaktionsparametern wie dem Druck, der Temperatur, der Rhodiumkonzentration und auch dem Reaktionsmedium abhängt, versteht sich von selbst, jedoch ist es für das erfindungsgemäße Verfahren nach den bisherigen Beobachtungen charakteristisch, daß die Mitverwendung des Cobaltkatalysators unter sonst gleichen Bedingungen jeweils zu höheren Ausbeuten an Ethylenglycol führt, verglichen mit der Arbeitsweise ohne den Cobaltkatalysator.

Für den Gesamtdruck empfielt sich ein Bereich von 300-3 000 bar, vorzugsweise 650-2 000 bar. Der Partialdruck des Kohlenmonoxids liegt hierbei vorzugsweise zwischen 20 und 80 %, besonders zwischen 30 und 60 % des Gesamtdruckes.

Gute Ergebnisse erzielt man bei Temperaturen von 180-280 °C, wobei der Bereich von 200-240 °C im allgemeinen zu bevorzugen ist.

Die Rhodium-Konzentration im Reaktionsmedium kann im Prinzip beliebig sein, da sie im wesentlichen nur einen Einfluß auf die Reaktionsgeschwindigkeit und damit auf die Raum-Zeit-Ausbeute hat. Befriedigende Raum-Zeit-Ausbeuten werden in der Regel im Konzentrationsbereich von 0,05-0,3 Gew.% Rh erzielt ; höhere Konzentrationen bringen keine nennenswerten wirtschaftlichen Vorteile mehr, und bei geringeren Konzentrationen — etwa herab bis zu 0,01 Gew.% — verlangsamt sich die Reaktion entsprechend.

Als Reaktionsmedium eignen sich im Prinzip alle inerten organischen Flüssigkeiten, also z. B. Kohlenwasserstoffe wie Toluol, Xylol und Cyclohexan, Ether wie Mono- und Di-Alkyl- und Arylether von Mono-, Di-, Tri- und Tetraethylenglykol, Alkohole wie Methanol, Ethanol, Propanol und Cyclohexanol und Sulfone wie Sulfolan.

Besonders gut geeignet sind offenkettige und cyclische Tetraalkylharnstoffe, Lactone sowie N-Alkyl- und N-Aryl-pyrrolidone. Unter diesen Lösungsmitteln haben sich N-Methylpyrrolidon, 1,5-Dimethylpyrrolid-2-on und 1,3-Dimethylimidazolid-2-on besonders bewährt.

Ferner empfiehlt es sich auch im vorliegenden Falle, die Reaktion in Gegenwart ein offenkettigen oder cyclischen tertiären Amins wie N-Methylmorpholin vorzunehmen, da derartige Verbindungen als Liganden in die Katalysatoren eintreten und deren Stabilität bekanntermaßen erhöhen. Die Menge dieser Verbindungen beträgt vorzugsweise etwa 1-50 mol pro Mol der Zentralatome Rh und Co.

Man kann die Reaktion nach den hierfür üblichen Techniken diskontinuierlich oder kontinuierlich vornehmen. Die Aufarbeitung der Reaktionsgemische erfolgt zweckmäßigerweise destillativ. Der hierbei anfallende katalisatorhaltige Rückstand kann zusammen mit den Lösungsmitteln für weitere Reaktionsansätze verwendet bzw. bei kontinuierlichem Betrieb wieder in die Synthesestufe zurückgeführt werden.

Als Verfahrensprodukte erhält man hauptsächlich Ethylenglycol sowie daneben Methanol, Ethanol und geringe Menge sonstiger Alkohole.

### Beispiel 1

Eine Lösung aus jeweils 100 ml 1,3-Dimethylimidazolid-2-on, 0,5 g $Rh(CO)_2$ · Acetylaceton (= 1,9 mmol Rh), 0,94 g (9,3 mmol) N-Methylmorpholin und $Co_2(CO)_8$ entsprechend einem Rh/Co-Molverhältnis q wurde 6 Stunden lang bei 240 C und 650 bar mit einem äquimolaren $CO/H_2$-Gemisch zur Reaktion gebracht.

Die gaschromatographische Analyse der Reaktionsgemische lieferte die in nachstehender Tabelle aufgeführten Werte.

| Versuch | q | Ausbeute in g an | | |
|---|---|---|---|---|
| | | Ethylenglycol | Methanol | Ethanol |
| zum Vergleich | | | | |
| a | 1 | 6,82 | 4,20 | 0,94 |
| b | 2,5 | 7,07 | 4,57 | 1,87 |
| c | 10 | 7,99 | 3,46 | 0,61 |
| d | ∞ (ohne Co) | 6,13 | 2,38 | 0,36 |
| erfindungsgemäß | | | | |
| e | 20 | 8,29 | 3,93 | 0,81 |
| f | 25 | 8,93 | 4,42 | 1,00 |
| g | 30 | 8,66 | 3,65 | 0,66 |
| h | 41 | 8,84 | 3,91 | 0,82 |
| i | 47 | 9,14 | 3,58 | 0,63 |

Die Ethylenglycolausbeuten bei den erfindungsgemäßen Versuchen (e)-(i) sind um rd · 4 bis 14 % höher als bei Versuch (c).

### Beispiel 2

Es wurden jeweils 15 ml einer Lösung aus 0,5 g (1,9 mmol Rh) $Rh(CO)_2$ · Acetylaceton, 0,94 g (9,3 mmol) N-Methylpyrrolidon, 100 ml 1,5-Dimethylpyrrolid-2-on und $Co_2(CO)_8$ entsprechend einem Rh/Co-Molverhältnis q 6 Stunden lang bei 230 °C und einem Druck 1 500 bar mit einem äquimolaren $CO/H_2$-Gemisch zur Reaktion gebracht.

Gemäß GC-Analyse enthielt das Reaktionsgemisch für den Fall q = 25 10,9 Gew.% Ethylenglycol und für den Fall q=1,4 nur 4,1 Gew.% Ethylenglycol.

### Beispiel 3

Unter den Bedingungen von Beispiel 1, jedoch mit 0,75 g (2,9 mmol Rh) der Rhodiumverbindung und 1,41 g N-Methylmorpholin bildeten sich für den Fall q = 22 12,85 g Ethylenglycol und für den Fall q = 1,2 nur 6,55 g.

**0 171 632**

**Patentanspruch**

Verfahren zur Herstellung von Ethylenglycol aus Kohlenmonoxid und Wasserstoff unter erhöhtem Druck und bei erhöhter Temperatur in Gegenwart von Rhodium enthaltenden Katalysatoren, dadurch gekennzeichnet, daß man zusätzlich zu den Rhodiumkatalysatoren Cobaltkatalysatoren verwendet, wobei das Molverhältnis von Rhodium zu Cobalt 20 : 1 bis 60 : 1 beträgt.

**Claim**

A process for the preparation of ethylene glycol from carbon monoxide and hydrogen under superatmospheric pressure and at an elevated temperature in the presence of a rhodium-containing catalyst, wherein a cobalt catalyst is used in addition to the rhodium catalyst, the molar ratio of rhodium to cobalt being from 20 : 1 to 60 : 1.

**Revendication**

Procédé de préparation de l'éthylène-glycol au départ de l'oxyde de carbone et de l'hydrogène, sous pression et à température accrues en présence de catalyseurs au rhodium, caractérisé en ce que l'on utilise, à côté d'un catalyseur au rhodium, un catalyseur au cobalt et ce avec un rapport molaire du rhodium au cobalt compris entre 20 : 1 et 60 : 1.